# EUROPEAN PATENT APPLICATION

(11) **EP 4 831 161 A1**
(43) Date of publication of application: **28.10.2026**
(21) Application number: 25172210.4
(22) Date of filing: 24.04.2025
(51) Int. Cl.: C07D 249/12, C07C 241/02, C07C 243/18

(54) **PROCESS FOR THE PREPARATION OF PROTHIOCONAZOLE AND INTERMEDIATES THEREOF**

(71) Applicant: ADAMA MAKHTESHIM LTD., 8410001 Beer Sheva (IL)
(72) Inventor: PEN, Liza, 8422730 Beer Sheva (IL); RUBINOV, Boris, 8450835 Beer Sheva (IL); GELMAN, Elijah, 8495300 Beer Sheva (IL); YEHOSHUA MAGAL, Shiran, 8410001 Beer Sheva (IL)
(74) Representative: ABG Intellectual Property Law, S.L.

(57) **Abstract**

The present invention is directed to a process for the preparation of 2-(1-chlorocycloprop-1-yl)-3-(2-chlorophenyl)-2-hydroxypropyl-1-hydrazine or a salt thereof by reacting 2-(1-chlorocyclopropyl)-2-(2-chlorobenzyl)oxirane with hydrazine in the presence of a solvent selected from the group consisting of C₂-C₁₂-dialkylsulfoxides, C₂-C₁₂-dialkylamides and mixtures thereof.

## Description

### FIELD OF THE INVENTION

The present invention relates to the field of organic synthesis, specifically to an improved method for the preparation of prothioconazole and its intermediate 2-(1-chloro-cycloprop-1-yl)-3-(2-chlorophenyl)-2-hydroxy-propyl-1-hydrazine.

### BACKGROUND PRIOR ART

Prothioconazole, 2-[(2RS)-2-(1-chlorocyclopropyl)-3-(2-chlorophenyl)-2-hydroxypropyl]-2H-1,2,4-triazole-3(4H)-thione, is a fungicide used to treat infected crops, first described in US 5,789,430. The structure is reproduced below:

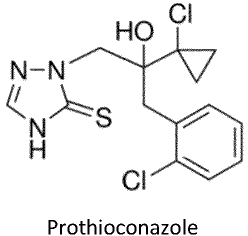

A key intermediate in the preparation of prothioconazole is 2-(1-chlorocycloprop-1-yl)-3-(2-chlorophenyl)-2-hydroxypropyl-1-hydrazine. Its preparation still presents many challenges as it can be deduced from the problems of the different strategies disclosed in the prior art. DE4030039A1 teaches the preparation of 2-(1-chloro-cyclopropyl)-1-(2-chlorophenyl)-3-(1,2,4-triazol-1-yl)propan-2-ol starting from 2-(1-chlorocyclopropyl)-2-(2-chlorobenzyl)oxirane using a two stage process. For the first stage, the oxirane is reacted with hydrazine in the presence of a solvent and, optionally, a phase transfer catalyst to provide 2-(1-chlorocycloprop-1-yl)-3-(2-chlorophenyl)-2-hydroxypropyl-1-hydrazine. The solvents used by DE4030039A1 for this reaction are alcohols and ethers, such as methanol, ethanol, n-propanol, isopropanol, n-butanol, diethyl ether, dioxane and tetrahydrofuran. For the second stage, DE4030039A1 teaches the same solvents. The hydrazine derivative is reacted with formamidine acetate in the presence of a phase transfer catalyst to provide 2-(1-chlorocyclopropyl)-1-(2-chlorophenyl)-3-(1,2,4-triazol-1-yl)propan-2-ol. The subsequent transformation of this intermediate into prothioconazole is achieved by reactions that involve the use of sulfur and, optionally butyllithium.

A similar teaching is found in WO 2021/074739. According to the description, the solvent used to obtain the 2-(1-chlorocycloprop-1-yl)-3-(2-chlorophenyl)-2-hydroxypropyl-1-hydrazine can be C₁-C₄ alcohols selected from methanol, ethanol, isopropanol and t -butanol; cyclohexanol. Other solvents, such as toluene, acetonitrile (ACN), N, N-dimethyl formamide (DMF) and sulfolane, are also mentioned. However, in the example shown on page 11 only n-butanol is used, which is partially soluble in water (about 73 g/L at 25°C), leaving the skilled person with the understanding that all other solvents mentioned should behave in a similar way and provide comparable results. The temperature taught for the reaction is 10°C to 110°C, and example 2 teaches a range of 60°C to 100°C.

US 2003/0013890 reports the preparation of 2-(1-chlorocycloprop-1-yl)-3-(2-chlorophenyl)-2-hydroxypropyl-1-hydrazine by reacting 2-(1-chlorocyclopropyl)-2-(2-chlorobenzyl)oxirane with hydrazine in the presence of an aromatic hydrocarbon (e.g. benzene, toluene, or xylene), optionally mixed with acetonitrile. The preferred temperature is between 60°C and 100°C (see WO0146158 in the same family). In example 1a) the reaction is indeed performed in a mixture of toluene and acetonitrile at 85°C. the comparative example 2a) is performed neat at 100°C. CN113666840 teaches the same reaction in the presence of toluene at 50-60°C. All examples are performed in toluene and polyethylene glycol dimethyl ether at 55-60°C.

In example 4 of CN111574397 the preparation of 2-(1-chlorocycloprop-1-yl)-3-(2-chlorophenyl)-2-hydroxypropyl-1-hydrazine is performed in methanol at 40-45°C with a yield of 80%. In example 9 the temperature is raised to 60-65°C to yield 90%.

WO 2022/107139 reports a two-phase reaction for the preparation of 2-(1-chlorocycloprop-1-yl)-3-(2-chlorophenyl)-2-hydroxypropyl-1-hydrazine in the presence of water and a second solvent, for example toluene.

### SUMMARY OF THE INVENTION

In the search for a more efficient process the inventors have designed reaction conditions for the preparation of 2-(1-chlorocycloprop-1-yl)-3-(2-chlorophenyl)-2-hydroxypropyl-1-hydrazine that provide higher yield and milder conditions.

The first aspect disclosed herein is therefore a process for the preparation of 2-(1-chlorocycloprop-1-yl)-3-(2-chlorophenyl)-2-hydroxypropyl-1-hydrazine or a salt thereof, the process comprising reacting 2-(1-chlorocyclopropyl)-2-(2-chlorobenzyl)oxirane and hydrazine in the presence of a solvent selected from the group consisting of C₂-C₁₂-dialkylsulfoxides, C₂-C₁₂-dialkylamides and mixtures thereof.

By using C₂-C₁₂-dialkylsulfoxides, C₂-C₁₂-dialkylamides or mixtures thereof high yields of 2-(1-chlorocycloprop-1-yl)-3-(2-chlorophenyl)-2-hydroxypropyl-1-hydrazine are obtained (see example 1 below with >95% yield). Additionally, milder conditions are possible, for example, the reaction can run efficiently at lower temperatures (e.g. below 50°C).

A further aspect is a process for the preparation of prothioconazole comprising
(i) preparing 2-(1-chlorocycloprop-1-yl)-3-(2-chlorophenyl)-2-hydroxypropyl-1-hydrazine or a salt thereof according to the method of the first aspect; and
(ii) transforming said 2-(1-chlorocycloprop-1-yl)-3-(2-chlorophenyl)-2-hydroxypropyl-1-hydrazine or salt thereof obtained in step (i) into prothioconazole.

### DETAILED DESCRIPTION OF THE INVENTION

### Preparation of 2-(1-chlorocycloprop-1-yl)-3-(2-chlorophenyl)-2-hydroxypropyl-1-hydrazine

The starting material used in the processes disclosed herein is 2-(1-chlorocyclopropyl)-2-(2-chlorobenzyl)oxirane, which can be obtained by methods already taught in the art, for example in DE 4030039A1 or in US 5,097,047. See for example columns 10 and 11 of US 5,097,047. For example, 2-(1-chlorocyclopropyl)-2-(2-chlorobenzyl)oxirane can be obtained by intra-cyclization of the corresponding hydroxy-chloride (1-Chloro-2-(1-chlorocyclopropyl)-3-(2-chlorophenyl)propan-2-ol) in the presence of an acid binder, as disclosed in paragraphs [0078]-[0081] of US 2001/0011138. Alternatively, the intramolecular cyclization can be performed using a base as taught in example 1.b) of DE4030039A1. The 2-(1-chlorocyclopropyl)-2-(2-chlorobenzyl)oxirane used as starting material can be used essentially pure or as a crude product from a previous synthetic step. The 2-(1-chlorocyclopropyl)-2-(2-chlorobenzyl)oxirane may contain small amounts of the corresponding open hydroxy-chloride (1-Chloro-2-(1-chlorocyclopropyl)-3-(2-chlorophenyl)propan-2-ol) or be in admixture with said hydroxy-chloride, which will also react with hydrazine to yield 2-(1-chlorocycloprop-1-yl)-3-(2-chlorophenyl)-2-hydroxypropyl-1-hydrazine. For example, the 2-(1-chlorocyclopropyl)-2-(2-chlorobenzyl)oxirane may contain up to 50 %w/w of the hydroxy-chloride, with respect to the total weight of 2-(1-chlorocyclopropyl)-2-(2-chlorobenzyl)oxirane plus hydroxy-chloride, for example, up to 40 %w/w or up to 30 %w/w or up to 20 %w/w or up to 10 %w/w or up to 5 %w/w or up to 3 %w/w, with respect to the total weight of 2-(1-chlorocyclopropyl)-2-(2-chlorobenzyl)oxirane plus hydroxy-chloride. Thus, the process of the invention may comprise reacting a mixture of 2-(1-chlorocyclopropyl)-2-(2-chlorobenzyl)oxirane, (1-Chloro-2-(1-chlorocyclopropyl)-3-(2-chlorophenyl)propan-2-ol) and hydrazine in the presence of a solvent selected from the group consisting of C2-C12-dialkylsulfoxides, C2-C12-dialkylamides and mixtures thereof, to provide 2-(1-chlorocycloprop-1-yl)-3-(2-chlorophenyl)-2-hydroxypropyl-1-hydrazine.

The C₂-C₁₂-dialkylsulfoxide used as solvent can be, for example a C₂-C₆-dialkylsulfoxide or a C₂-C₄-dialkylsulfoxide. Examples of said C₂-C₁₂-dialkylsulfoxides are dimethylsulfoxide (DMSO) or sulfolane. The C₂-C₁₂-dialkylamide can be, for example, a C₂-C₈-dialkylamide or a C₂-C₅-dialkylamide. Examples of C₂-C₁₂-dialkylamides are dimethylacetamide (DMA), dimethylformamide (DMF), N-methylpyrrolidine (NMP) or mixtures thereof. The C₂-C₁₂-dialkylsulfoxide and/or the C₂ -C₁₂-dialkylamide can be cyclic (e.g. sulfolane or NMP) or acyclic (e.g. DMSO, DMA or DMF).

Hydrazine can be used in any suitable form, typically hydrazine hydrate, and may be used as a solution in water of various concentrations. The hydrazine is typically used in molar excess with respect to the 2-(1-chlorocyclopropyl)-2-(2-chlorobenzyl)oxirane. The molar proportion between the hydrazine and 2-(1-chlorocyclopropyl)-2-(2-chlorobenzyl)oxirane is typically comprised between 1:1 and 20:1 or between 1:1 and 10:1, for example, between 3:1 and 20:1, or between 15:1 and 10:1, preferably, between 8:1 and 7:1. Said excess of hydrazine can be recycled into the reaction. If no recycling is desired, the process can function even at lower molar excesses of hydrazine, for example, the molar proportion between the hydrazine and 2-(1-chlorocyclopropyl)-2-(2-chlorobenzyl)oxirane can be comprised between 1:1 and 5:1, or between 1:1 to 4:1 or between 1.05:1 and 3:1.

The process uses C₂-C₁₂-dialkylsulfoxides , C₂-C₁₂-dialkylamides or mixtures thereof as solvent, wherein any amount of water added as part of the hydrazine is dissolved. Therefore, the process can be performed in the absence of a phase transfer catalyst. Neither does the reaction require a metal catalyst or any kind of catalyst.

The reaction typically proceeds at atmospheric pressure, but other pressures can be used. Temperature is typically required to proceed to completion in a reasonable time and the reaction is usually maintained at reflux, typically at a temperature comprised between 20°C and 150°C, for example between 40°C and 150°C, for example between 20°C and 100°C, for example between 50°C and 95°C. The reaction may however proceed at lower temperatures, for example at a temperature below 60°C or below 55°C or below 50°C. For example, the inventors have found that in combination with C₂ -C₁₂-dialkylsulfoxides, C₂-C₁₂-dialkylamides or mixtures thereof the reaction temperature can be comprised between 20°C and 60°C, for example, between 25°C and 55°C or between 30°C and 50°C or between 30°C and less than 50°C.

Thus, the 2-(1-chlorocyclopropyl)-2-(2-chlorobenzyl)oxirane can be reacted with hydrazine in the presence of a solvent selected from the group consisting of C₂-C₁₂-dialkylsulfoxides at a temperature comprised between 20°C and 60°C, preferably between 30°C and 50°C, more preferably between 30°C and less than 50°C.

Alternatively, the 2-(1-chlorocyclopropyl)-2-(2-chlorobenzyl)oxirane can be reacted with hydrazine in the presence of a solvent selected from the group consisting of C₂-C₁₂-dialkylamides at a temperature comprised between 20°C and 60°C, preferably between 30°C and 50°C, more preferably between 30°C and less than 50°C.

Alternatively, the 2-(1-chlorocyclopropyl)-2-(2-chlorobenzyl)oxirane can be reacted with hydrazine in the presence of a solvent selected from the group consisting of C₂-C₁₂-dialkylamides at a temperature comprised between 20°C and 60°C, preferably between 30°C and 50°C, more preferably between 30°C and less than 50°C, wherein said C₂-C₁₂-dialkylamide is selected from the group consisting of dimethylacetamide (DMA), dimethylformamide (DMF), N-metylpyrrolidine (NMP) and mixtures thereof.

Once the reaction is complete, the reaction mass can proceed to workup in the usual manner. For example, a solvent insoluble with C₂-C₁₂-dialkylsulfoxides, C₂-C₁₂-dialkylamides or mixtures thereof can be added to extract 2-(1-chlorocycloprop-1-yl)-3-(2-chlorophenyl)-2-hydroxypropyl-1-hydrazine. The unreacted hydrazine will remain in the C₂-C₁₂-dialkylsulfoxide, C₂-C₁₂-dialkylamide or mixture thereof and 2-(1-chlorocycloprop-1-yl)-3-(2-chlorophenyl)-2-hydroxypropyl-1-hydrazine will be extracted into the solvent, typically an aliphatic hydrocarbon or an aromatic hydrocarbon (e.g. toluene). This separation can be done at a temperature comprised between 20°C and 80°C, preferably between 40°C and 70°C.

The hydrazine can be directly recycled for subsequent reactions, especially when a large excess is used, optionally after being mixed with fresh hydrazine. For example, the process can use a molar proportion between the hydrazine and 2-(1-chlorocyclopropyl)-2-(2-chlorobenzyl)oxirane comprised between 3:1 and 20:1 or between 4:1 and 20:1, wherein the excess of hydrazine is recycled for subsequent reactions.

The remaining toluene containing 2-(1-chlorocycloprop-1-yl)-3-(2-chlorophenyl)-2-hydroxypropyl-1-hydrazine can be optionally washed, for example, at least one time with a neutral or basic solution, and then be used directly in the next step towards prothioconazole, which is typically the preparation of 2-(2-(1-Chlorocyclopropyl)-3-(2-chlorophenyl)-2-hydroxypropyl)-1,2,4-triazolidine-3-thione.

2-(1-chlorocycloprop-1-yl)-3-(2-chlorophenyl)-2-hydroxypropyl-1-hydrazine is sufficiently stable in toluene to be used in subsequent steps without any change of solvent (no evaporation is necessary), which is a very efficient strategy. This solution can be obtained at different concentrations, typically between 1 %w/w and 99 %w/w of the 2-(1-chlorocycloprop-1-yl)-3-(2-chlorophenyl)-2-hydroxypropyl-1-hydrazine in toluene, for example between 5 %w/w and 95 %w/w, preferably between 10 %w/w and 80 %w/w, preferably between 12 %w/w and 60 %w/w, more preferably between 15 %w/w and 50 %w/w, preferably between 15 %w/w and 40 %w/w, even more preferably between 20 %w/w and 30 %w/w of 2-(1-chlorocycloprop-1-yl)-3-(2-chlorophenyl)-2-hydroxypropyl-1-hydrazine in toluene. Therefore, 2-(1-chlorocyclopropyl)-2-(2-chlorobenzyl)oxirane can be transformed into 2-(1-chlorocycloprop-1-yl)-3-(2-chlorophenyl)-2-hydroxypropyl-1-hydrazine, which can undergo further transformations

towards prothioconazole without the need to isolate 2-(1-chlorocycloprop-1-yl)-3-(2-chlorophenyl)-2-hydroxypropyl-1-hydrazine.

Methods to transform 2-(1-chlorocycloprop-1-yl)-3-(2-chlorophenyl)-2-hydroxypropyl-1-hydrazine into prothioconazole are known in the art, for example, in US 2001/0011138 or US 2002/0026058.

As way of example, the process may comprise
(i) preparing 2-(1-chlorocycloprop-1-yl)-3-(2-chlorophenyl)-2-hydroxypropyl-1-hydrazine or a salt thereof, by reacting 2-(1-chlorocyclopropyl)-2-(2-chlorobenzyl)oxirane with hydrazine in the presence of a solvent selected from the group consisting of C₂-C₁₂-dialkylsulfoxides, C₂-C₁₂-dialkylamides and mixtures thereof; and
(ii) reacting said 2-(1-chlorocycloprop-1-yl)-3-(2-chlorophenyl)-2-hydroxypropyl-1-hydrazine or a salt thereof with formaldehyde and a thiocyanate of formula X-SCN, wherein X is an alkaline cation or ammonium, in the presence of an acid (e.g. phosphoric acid, HCl, sulfuric, formic acid, acetic acid) to provide the 2-(2-(1-Chlorocyclopropyl)-3-(2-chlorophenyl)-2-hydroxypropyl)-1,2,4-triazolidine-3-thione.

The 2-(1-chlorocycloprop-1-yl)-3-(2-chlorophenyl)-2-hydroxypropyl-1-hydrazine obtained in step (i) can be crude in admixture with another solvent (e.g. toluene) before being submitted to step (ii). Optionally, the mixture of 2-(1-chlorocycloprop-1-yl)-3-(2-chlorophenyl)-2-hydroxypropyl-1-hydrazine in solvent resulting from step (i) is washed with an aqueous solution before use.

Thus, the process for the preparation of 2-(2-(1-Chlorocyclopropyl)-3-(2-chlorophenyl)-2-hydroxypropyl)-1,2,4-triazolidine-3-thione may comprise
(i) reacting 2-(1-chlorocyclopropyl)-2-(2-chlorobenzyl)oxirane with hydrazine in the presence of a solvent selected from the group consisting of C₂-C₁₂-dialkylsulfoxides, C₂-C₁₂-dialkylamides and mixtures thereof, to provide after workup a mixture comprising 2-(1-chlorocycloprop-1-yl)-3-(2-chlorophenyl)-2-hydroxypropyl-1-hydrazine in a solvent, preferably a water-insoluble solvent (e.g. toluene); and
(ii) reacting said mixture comprising 2-(1-chlorocycloprop-1-yl)-3-(2-chlorophenyl)-2-hydroxypropyl-1-hydrazine in a solvent, with formaldehyde and a thiocyanate of formula X-SCN, wherein X is an alkaline cation or ammonium, in the presence of an acid (e.g. phosphoric acid, HCl, sulfuric, formic acid, acetic acid) to provide 2-(2-(1-Chlorocyclopropyl)-3-(2-chlorophenyl)-2-hydroxypropyl)-1,2,4-triazolidine-3-thione.

Step (ii) typically proceeds at atmospheric pressure, but other pressures can be used. Temperature is typically required to proceed in a reasonable time and the reaction is usually maintained at a temperature comprised between 20°C and 100°C, for example between 30°C and 80°C. The formaldehyde can be employed as paraformaldehyde, as gaseous formaldehyde or as Formalin solution (aqueous formaldehyde solution). Preference is given to using Formalin solution. The preferred thiocyanate is sodium thiocyanate. Both, formaldehyde and the thiocyanate are typically used in excess, for example from 1 to 2 equivalents of formaldehyde and/or from 1 to 2 equivalents of thiocyanate for every mol of hydrazine derivative of Formula (I). Typically, up to 1.5 equivalents of formaldehyde are used, preferably, between 1 and 1.4 or between 1 and 1.2.

The 2-(2-(1-Chlorocyclopropyl)-3-(2-chlorophenyl)-2-hydroxypropyl)-1,2,4-triazolidine-3-thione obtained can be purified through the usual processes, such as chromatographic purification or recrystallization. The order of addition of formaldehyde, thiocyanate and acid is irrelevant.

In order to provide prothioconazole, 2-(2-(1-Chlorocyclopropyl)-3-(2-chlorophenyl)-2-hydroxypropyl)-1,2,4-triazolidine-3-thione must be oxidized or aromatized. This can be done by following processes already disclosed in the prior art, such as for example, WO 2019/123368 A1 (see pages 9 through 13). Exemplary methods to perform the oxidation involve the aromatization in the presence of an oxidizing agent in a suitable solvent or mixture of solvents. Alternatively, aromatization can be acid catalyzed in the presence of an oxidizing agent in a suitable solvent or mixture of solvents. Acids can be selected from the group consisting of hydrochloric acid, hydrobromic acid, acetic acid, benzoic acid, substituted benzoic acid, trifluoroacetic acid, and formic acid. Acids can also be Lewis acids such as, aluminum chloride, stannous chloride, stannic chloride, titanium tetrachloride, boron trifluoride diethyl etherate, boron THF complex, or zinc chloride. Oxidizing agents can be selected from the group consisting of hydrogen peroxide, m-chloro perbenzoic acid (ra-CPBA), oxone, tert- butyl hydrogen peroxide (TBHP), copper sulphate, sodium nitrite, t-butyl nitrite, Ferric Chloride (III) and tert- butyl nitrite.

### EXAMPLES

### Example 1: preparation of 2-(1-chlorocycloprop-1-yl)-3-(2-chlorophenyl)-2-hydroxypropyl-1-hydrazine

Into 1L reactor, equipped with overhead stirrer, add 400 gr DMSO. Add 200 gr of Hydrazine Hydrate 100% (4 mol) and stir under Nitrogen flow for 0.5-1 h. Then feed 180 gr 2-(1-chlorocyclopropyl)-2-(2-chlorobenzyl)oxirane (0.74 mol) over 1 h, keeping reactor temperature up to 40-45°C. Keep the reaction mixture at 40-45°C for additional 5 h to reach the end of reaction condition (less than 0.1 w/w% of 2-(1-chlorocyclopropyl)-2-(2-chlorobenzyl)oxirane remaining). Add 400 gr of toluene, and stir for 0.5 h at 40-45°C. Separate the phase to obtain upper Toluene phase rich with 2-(1-chlorocycloprop-1-yl)-3-(2-chlorophenyl)-2-hydroxypropyl-1-hydrazine, and lower DMSO phase rich with Hydrazine Hydrate. Add an additional portion of 200 gr of Toluene to DMSO phase from previous separation, mix for 0.5 h at 40-45°C, and separate the phases. Combine both Toluene phases and wash with 400 gr of fresh water for 0.5 h at 40-45°C. Separate phases to obtain upper Toluene phase that contains 25-27 w/w% of M275. Yield on 2-(1-chlorocycloprop-1-yl)-3-(2-chlorophenyl)-2-hydroxypropyl-1-hydrazine: 95-98%.

### Example 2: Comparative example

Into 1L reactor, equipped with overhead stirrer, add 360 gr Methanol. Add 200 gr of Hydrazine Hydrate 100% (4 mol) and stir under Nitrogen flow for 0.5-1 h. Then feed 180 gr 2-(1-chlorocyclopropyl)-2-(2-chlorobenzyl)oxirane (0.74 mol) over 1.5 h, keeping reactor temperature up to 40-45°C. Keep the reaction mixture at 40-45°C for additional 4-5 h to reach the end of reaction condition (less than 0.1 w/w% of 2-(1-chlorocyclopropyl)-2-(2-chlorobenzyl)oxirane remaining). Distill the methanol under reduced pressure, while maintaining reactor temperature at 40-45°C. Add 500 gr of toluene and stir for 0.5 h at 40-45°C. Separate the phase to obtain upper Toluene phase rich with 2-(1-chlorocycloprop-1-yl)-3-(2-chlorophenyl)-2-hydroxypropyl-1-hydrazine, and lower aqueous phase rich with Hydrazine Hydrate. Having distilled the methanol, only one extraction is necessary. Wash twice with 200 gr of fresh water for 0.5 h at 40-45°C. Separate phases to obtain upper Toluene phase that contains 25-27 w/w% of M275. Yield on 2-(1-chlorocycloprop-1-yl)-3-(2-chlorophenyl)-2-hydroxypropyl-1-hydrazine: 81-85%.

## Claims

1. A process for the preparation of 2-(1-chlorocycloprop-1-yl)-3-(2-chlorophenyl)-2-hydroxypropyl-1-hydrazine or a salt thereof, the process comprising reacting 2-(1-chlorocyclopropyl)-2-(2-chlorobenzyl)oxirane with hydrazine in the presence of a solvent selected from the group consisting of C₂-C₁₂-dialkylsulfoxides, C₂-C₁₂-dialkylamides and mixtures thereof.

2. The process according to claim 1, wherein the solvent is a C₂-C₆-dialkylsulfoxide.

3. The process according to any of the previous claims, wherein the solvent is dimethylsulfoxide.

4. The process according to claim 1, wherein the solvent is a C₂-C₅-dialkylamide.

5. The process according to claim 4, wherein the solvent is selected from the group consisting of dimethylacetamide (DMA), dimethylformamide (DMF), N-metylpyrrolidine (NMP) and mixtures thereof.

6. The process according to any of the previous claims, wherein the reaction is done in the absence of a phase transfer catalyst.

7. The process according to any of the previous claims, wherein the molar proportion between hydrazine and 2-(1-chlorocyclopropyl)-2-(2-chlorobenzyl)oxirane is comprised between 1:1 and 20:1.

8. The process according to any of the previous claims, wherein the molar proportion between hydrazine and 2-(1-chlorocyclopropyl)-2-(2-chlorobenzyl)oxirane is comprised between 1:1 and 6:1.

9. The process according to any of the previous claims, wherein the reaction temperature is below 60°C.

10. The process according to any of the previous claims, wherein the reaction temperature is comprised between 25°C and 55°C.

11. The process according to claim 1, wherein 2-(1-chlorocyclopropyl)-2-(2-chlorobenzyl)oxirane is reacted with hydrazine in the presence of a solvent selected from the group consisting of C₂-C₁₂-dialkylsulfoxides at a temperature comprised between 20°C and 60°C.

12. The process according to claim 1, wherein 2-(1-chlorocyclopropyl)-2-(2-chlorobenzyl)oxirane is reacted with hydrazine in the presence of a solvent selected from the group consisting of C₂-C₁₂-dialkylamides at a temperature comprised between 20°C and 60°C.

13. The process according to claim 1, wherein 2-(1-chlorocyclopropyl)-2-(2-chlorobenzyl)oxirane is reacted with hydrazine in the presence of a solvent selected from the group consisting of C₂-C₁₂-dialkylamides at a temperature comprised between 20°C and 60°C, wherein said C₂-C₁₂-dialkylamide is selected from the group consisting of dimethylacetamide (DMA), dimethylformamide (DMF), N-metylpyrrolidine (NMP) and mixtures thereof.

14. The process according to any of the previous claims, comprising the additional step of transforming said 2-(1-chlorocycloprop-1-yl)-3-(2-chlorophenyl)-2-hydroxypropyl-1-hydrazine or salt thereof into prothioconazole.

15. A process for the preparation of prothioconazole comprising
(i) preparing 2-(1-chlorocycloprop-1-yl)-3-(2-chlorophenyl)-2-hydroxypropyl-1-hydrazine or a salt thereof according to a method as defined in any of claims 1 to 14; and
(ii) transforming the 2-(1-chlorocycloprop-1-yl)-3-(2-chlorophenyl)-2-hydroxypropyl-1-hydrazine or salt thereof obtained in step (i) into prothioconazole.
